# FASCICULE DE BREVET EUROPEEN

(11) **EP 0 675 977 B1**
(45) Date de publication et mention de la délivrance du brevet: **30.10.1996**
(21) Numéro de dépôt: 94900206.7
(22) Date de dépôt: 18.11.1993
(51) Int. Cl.: D04H 13/00

(54) **MATERIAU NON-TISSE COMPOSITE ET SON APPLICATION A TOUT ARTICLE D'HYGIENE ABSORBANT**
VERBUNDVLIESSTOFF UND SEINE ANWENDUNG FÜR SAUGFÄHIGE HYGIENISCHE PRODUKTE
NON-WOVEN COMPOSITE MATERIAL AND APPLICATION TO ANY ABSORBANT SANITARY ARTICLE

(30) Priorité: 20.11.1992 FR 9213998
(43) Date de publication de la demande: 11.10.1995
(73) Titulaire: PEAUDOUCE, F-59126 Linselles (FR)
(72) Inventeur: KOCZAB, Jean-Pierre, F-59910 Bondues (FR)
(74) Mandataire: Casalonga, Axel
(86) Numéro de dépôt international: FR9301132
(87) Numéro de publication internationale: WO9412714

(56) Documents cités:
- EP-A- 0 151 018
- EP-A- 0 232 729
- EP-A- 0 313 800
- DE-A- 3 029 315
- FR-A- 2 169 348
- US-A- 4 985 279

## Description

L'invention concerne, d'une manière générale, un nouveau matériau non-tissé, qui lorsqu'il est utilisé comme voile ou feuille de surface ou en complément du voile de surface comme bande dans la zone d'entre-jambes dans un article d'hygiène absorbant, tel qu'une couche-culotte ou garniture pour incontinent, permet une meilleure isolation de la peau de l'utilisateur de la partie absorbante de l'article d'hygiène.

D'une manière générale, les articles d'hygiène absorbants, tels que des couches-culottes et garnitures pour incontinents, comprennent une couche externe en matériau imperméable aux fluides corporels un coussin ou matelas de matériau absorbant et un voile ou feuille de surface perméable aux fluides corporels, tels que l'urine, de dimension et de forme analogues à celles à la couche externe imperméable de l'article. Ce voile de surface perméable aux fluides corporels a pour but d'isoler la peau du coussin absorbant humidifié. Par conséquent, le voile de surface doit avoir un degré de douceur convenable et assurer une isolation voulue entre la peau et le coussin absorbant. Le coussin absorbant a pour fonction d'absorber les fluides et par conséquent doit présenter une vitesse d'absorption importante ainsi qu'une grande capacité d'absorption. Un coussin absorbant particulièrement efficace est décrit dans le document EP-A-0 232 729. Ce coussin ou matelas absorbant se compose d'une nappe de fibres absorbantes longues doublée sur ses faces d'une couche de ouate de cellulose. La nappe doublée par les couches de ouate est aiguilletée depuis les deux faces.

Un matelas particulièrement efficace décrit dans cette demande de brevet EP-A-0 232 729 comprend deux nappes de fibres longues, une première nappe de fibres absorbantes et une deuxième nappe de fibres non absorbantes, entre lesquelles sont disposées des particules de matière superabsorbante, la nappe de fibres non absorbantes étant doublée extérieurement par une couche de non-tissé et la nappe de fibres absorbantes étant doublée extérieurement d'une couche de ouate de cellulose. L'ensemble est lié par aiguilletage depuis les deux faces.

Dans les articles d'hygiène absorbants de tels matelas ou coussins absorbants sont recouverts par un voile de surface généralement en matériau non-tissé qui a pour but d'isoler la peau du coussin absorbant et qui doit assurer un contact agréable avec la peau et l'isolation voulue avec le coussin absorbant. Ces voiles ou feuilles de surface doivent présenter comme propriétés essentielles un contact agréable avec la peau, une vitesse de traversée par les fluides corporels importante et constituer une barrière vis-à-vis du remouillage.

Le document FR-A-2 588 285 décrit un textile non-tissé multicouche ayant au moins deux couches de voile non-tissé, l'une des couches étant formée de fibres de section transversale bilobée et l'autre couche étant formée de fibres de section transversale trilobée. Chaque couche de voile est de préférence obtenue par la technique de liaison au filage (spun bonded) et les deux couches de voile sont réunies pour former le non-tissé multicouche par liaison thermique en des zones compactées et discontinues.

Le document WO 87/07117 décrit un article d'hygiène absorbant comprenant un corps absorbant entouré d'une enveloppe. Cette enveloppe ou voile de surface est constitué de deux couches en matériau non-tissé. La première couche de matériau non-tissé, en contact avec la peau de l'utilisateur, est constituée d'une mince couche de tissu fibreux lié au filage en un matériau hydrophobique, et la seconde couche en contact avec le corps absorbant est une couche fibreuse hydrophobique de tissu de fibres liée par fusion, de construction similaire à la première couche. Ces deux couches de voile de surface ne sont pas liées entre elles dans la zone destinée à venir en contact avec le corps de l'utilisateur.

Le document WO 88/05269 concerne un voile de surface pour un article absorbant jetable composé d'au moins deux couches de non-tissé qui peuvent être identiques ou différentes et qui sont réunies par des lignes d'adhésif formant un motif ouvert.

La présente invention a donc pour but de fournir un matériau non-tissé composite présentant un degré de douceur voulu, une vitesse de traversée par les liquides et une résistance au remouillage améliorées.

La présente invention a également pour but de fournir un article d'hygiène absorbant tel que des couches-culottes et garnitures pour incontinents comportant un voile de surface formé d'un tel matériau non-tissé composite.

La présente invention a encore pour but de fournir un article d'hygiène absorbant tel que des couches-culotte et garnitures pour incontinents comportant en complément d'un voile de surface en non-tissé classique, une bande dans la zone d'entre-jambes formée du matériau non-tissé composite selon l'invention.

Selon la présente invention on réalise un matériau non-tissé composite caractérisé en ce qu'il comprend au moins une première couche constituée par un non-tissé et, sur cette première couche, une nappe de fibres de type cardé, la nappe de fibres de type cardé étant liée à la couche de base par aiguilletage.

Dans une autre réalisation de la présente invention, le matériau non-tissé composite comprend une première couche en un non-tissé, une nappe de fibres de type cardé et une seconde couche en un non-tissé de grammage inférieur à la première couche de non-tissé, la nappe de fibres de type cardé étant disposée entre la première et la deuxième couches de non-tissé, l'ensemble étant lié par aiguilletage.

Le matériau composite selon l'invention présente un temps de transpercement par les fluides corporels plus court et un degré d'isolation meilleur que les non-tissés antérieurs utilisés jusqu'à présent pour former les voiles de surface et bande de zone d'entre-jambes d'articles d'hygiène absorbants.

Selon la présente invention on réalise également un article d'hygiène absorbant tel qu'une couche-culotte qui comprend une couche externe en matériau imperméable, un matelas absorbant et sur ce matelas absorbant un voile de surface constitué par le matériau composite selon l'invention. Lorsque le voile de surface est constitué par le matériau composite selon l'invention comprenant une couche de non-tissé et la nappe de fibres de type cardé, la nappe de type cardé constitue la couche la plus externe du voile, c'est-à-dire la couche directement en contact avec le coussin absorbant, cependant que la couche de non-tissé constitue la couche la plus interne du voile de surface qui sera en contact avec le corps de l'utilisateur. Lorsque le voile de surface est constitué par le matériau composite selon l'invention comprenant une nappe de fibres cardée entre deux couches de non-tissé, la couche de non-tissé de plus faible grammage constitue la couche la plus externe du voile de surface, c'est-à-dire la couche directement en contact avec le coussin absorbant, et la couche de non-tissé de grammage supérieur constitue la couche la plus interne du voile, qui sera directement en contact avec le corps de l'utilisateur.

L'invention fournit également un article d'hygiène absorbant tel qu'une couche-culotte, qui comprend une couche externe en matériau imperméable aux fluides corporels, un matelas absorbant, un voile de surface en non-tissé et une bande dans la zone d'entre-jambes constituée du matériau composite selon l'invention. La bande dans la zone d'entre-jambes peut être disposée soit sur le voile de surface en non-tissé classique soit entre ce voile en non-tissé classique et le coussin absorbant. Comme précédemment, lorsque l'on utilise le matériau composite selon l'invention constitué d'une couche de non-tissé et de la nappe en fibres de type cardé, cette nappe en fibres de type cardé constituera la couche la plus externe de la bande de la zone d'entre-jambes cependant que la couche de non-tissé constituera la couche la plus interne de la bande de la zone d'entre-jambes. Egalement dans le cas où la bande de la zone d'entre-jambes est constituée par le matériau composite selon l'invention comportant une nappe de fibres de type cardé comprise entre deux couches de non-tissé, la couche de non-tissé de plus faible grammage constituera la couche la plus externe de la bande de la zone d'entre-jambes, cependant que la couche de non-tissé de grammage supérieur constituera la couche la plus interne de cette bande.

La suite de la description se réfère aux figures annexées qui représentent respectivement :
figure 1, une première réalisation du matériau non-tissé composite selon l'invention;
figure 2, une seconde réalisation du matériau non-tissé composite selon l'invention;
figure 3, une vue de dessus, en partie arrachée, d'un article d'hygiène absorbant. tel qu'une couche-culotte, comportant un voile de surface constitué du matériau composite de la figure 1;
figure 4, une vue en coupe faite suivant la ligne IV-IV de la figure 3;
figure 5, une vue de dessus, en partie arrachée, d'un article d'hygiène absorbant, tel qu'une couche-culotte comportant une bande de zone d'entre-jambes constituée du matériau composite de la figure 1;
figure 6, une vue en coupe faite suivant la ligne VI-VI de la figure 5;
figure 7 une vue de dessus, en partie arrachée, d'un article d'hygiène absorbant tel qu'une couche-culotte, comportant un voile de surface en matériau non-tissé composite selon la figure 2;
figure 8, une vue en coupe faite suivant la ligne VIII-VIII de la figure 7;
figure 9, une vue de dessus, en partie arrachée, d'un article d'hygiène absorbant tel qu'une couche-culotte, comportant une bande de zone d'entre-jambes constituée du matériau non-tissé composite de la figure 2; et
figure 10, une vue en coupe faite suivant la ligue X-X de la figure 9.

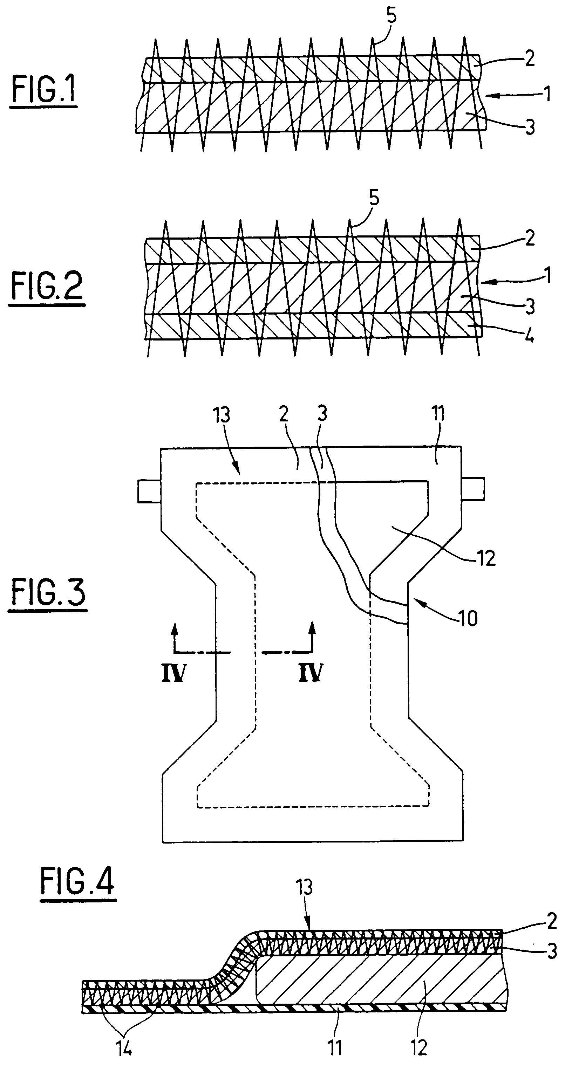

En se référant à la figure 1, on a représenté une première réalisation du matériau non-tissé composite 1 selon l'invention. Ce matériau comprend une première couche 2 en un non-tissé perméable aux fluides corporels, tels que l'urine, et sur cette première couche en non-tissé, une nappe de fibres de type cardé 3, formant matelas, perméable aux fluides corporels, la nappe de fibres de type cardé étant liée à la première couche en non-tissé par un aiguilletage 5 à partir des deux faces.

On peut utiliser pour la première couche en non-tissé, tous types de non-tissé classiques, par exemple, en fibres textiles naturelles ou synthétiques, telles que les fibres de cellulose, de viscose, de polyester, de polyéthylène, de polypropylène, de nylon, ou de copolymères éthylène-propylène. De plus la première couche de non-tissé 2 peut être formée par des fibres de même nature ou de nature différente. Egalement, cette couche de non-tissé peut être constituée de fibres de même denier ou de deniers différents. Cette première couche de non-tissé peut être constituée d'un non-tissé fabriqué par tous procédés classiques tels que, par exemple, liaison au filage (spun bonded), liaison thermique, liaison chimique, liaison par soufflage d'air, laçage au filage (spun lace). De préférence cette première couche de non-tissé 2 a un grammage compris entre 10 et 30 g/m².

La nappe de fibres de type cardé 3, perméable aux fluides, peut être constituée par des fibres textiles synthétiques telles que du polyester, du polyéthylène, du polypropylène, du nylon ou des copolymères d'éthylène-propylène. On recommande tout particulièrement les fibres de polyester pour leurs caractéristiques de résilience et de confort. On peut également utiliser des fibres profilées, par exemple des fibres bi- ou trilobées. L'utilisation de telles fibres permet le transport ou la diffusion des fluides du fait de leur forme propre. La nappe de fibres de type cardé peut être constituée de fibres de même nature ou de mélange de fibres en fonction du type de barrière voulu. Egalement la nappe de fibres de type cardé 3 peut être constituée de fibres de deniers (mesure de la grosseur du diamètre des fibres) identiques ou différents, le denier des fibres de cette nappe étant généralement compris entre 3 et 13. En général, la nappe de fibres de type cardé a un grammage compris entre 20 et 60 g/m².La nappe de fibres de type cardé 3 est obtenue par les techniques de cardage ou de pseudocardage bien connues.

De préférence, la première couche de non-tissé 2 et la nappe de fibres de type cardé 3 sont liées par aiguilletage à partir des deux faces du composite. Bien évidemment, la densité d'aiguilletage et le choix du denier des fibres déterminent le gonflant (épaisseur du matériau non-tissé). On utilise généralement une densité d'aiguilletage de 10 à 100 coups d'aiguille par cm² et par face. On peut utiliser pour l'aiguilletage un procédé d'aiguilletage à simple frappe ou double frappe. Le matériau non-tissé composite obtenu a en général une densité comprise entre 50 et 300kg/m³, et la couche de nappe de fibres de type cardé 3 a en général une épaisseur supérieure à celle de la première couche en non-tissé 2.

En se référant maintenant à la figure 2, on a représenté une autre réalisation d'un matériau non-tissé multicouche selon la présente invention. Le matériau comprend une première couche en non-tissé 2 analogue à la couche en non-tissé 2 de la réalisation de la figure 1. Sur cette première couche de non-tissé 2 se trouve une nappe de fibres de type cardé 3 également analogue à la nappe de fibres de type cardé de la figure 1. Sur la surface supérieure de la nappe de fibres de type cardé 3 se trouve une seconde couche de non-tissé 4. Cette seconde couche de non-tissé 4 est analogue à la première couche de non-tissé 2 sauf en ce qui concerne son grammage. Cette seconde couche de non-tissé doit avoir un grammage inférieur à celui de la première couche de non-tissé 2, et de préférence inférieur à 20 g/m², par exemple de 17 g/m² et mieux encore inférieur ou égal à 15 g/m². Comme pour le matériau non-tissé composite de la figure 1, les couches du matériau non-tissé composite de la figure 2 sont réunies par aiguilletage de la même façon.

Les matériaux non tissés composites selon l'invention s'avèrent particulièrement utiles comme voiles de surface ou bande de zone d'entre-jambes dans des articles d'hygiène absorbants tels que des couches-culottes ou des garnitures pour incontinents.

En se référant aux figures 3 et 4 on a représenté un article d'hygiène absorbant 10, tel qu'une couche-culotte, comprenant une couche externe 11 en un matériau souple imperméable aux fluides corporels sur laquelle est fixé un matelas ou coussin absorbant 12 perméable aux fluides corporels de dimension inférieure à la couche externe. Le coussin absorbant 12 est fixé à la couche imperméable 11 par tout moyen classique tel que par collage. Sur ce coussin absorbant 12 se trouve un voile de surface 13 perméable aux fluides corporels de dimension analogue à celle de la couche externe 11. Le voile de surface 13 est lié sur le pourtour du coussin absorbant 12 à la couche externe 11 par tout moyen tel que par collage.Comme cela est bien connu dans la technique, la couche externe 11, le matelas absorbant 12 et le voile de surface 13 ont la forme d'un sablier comprenant deux parties d'extrémité opposées relativement larges réunies par une partie ou zone d'entre-jambes plus étroite. Comme le montre les figures 3 et 4, le voile de surface est constitué du matériau non-tissé composite représenté à la figure 1 et qui comprend une première couche de non-tissé 2 et une nappe de fibres de type cardé 3. Comme le montrent les figures, la nappe de type cardé 3 est directement placée au-dessus du coussin absorbant, cependant que la couche de non-tissé 2 constitue la couche la plus interne du voile destinée à entrer en contact avec le corps de l'utilisateur.

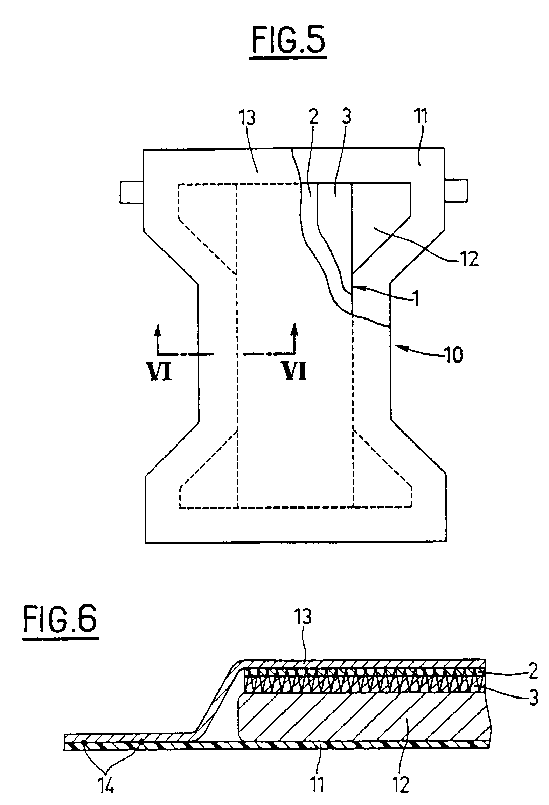

Lorsqu'il est utilisé en complément d'un voile de surface classique, le matériau composite selon l'invention est de préférence utilisé sous forme d'une bande de zone d'entre-jambes ayant une largeur semblable à celle de la zone d'entre-jambes du coussin et une longueur analogue à celle de ce coussin. Dans cette réalisation, la bande de zone d'entre-jambes constituée du matériau selon l'invention est disposée soit au-dessus du voile de surface classique soit entre ce voile de surface classique et le coussin. Sur les figures 5 et 6 on a représenté une telle bande de zone d'entre-jambes constituée du matériau composite représenté à la figure 1. Dans cette réalisation, l'article d'hygiène absorbant, tel qu'une couche-culotte, comprend une couche externe en un matériau imperméable aux fluides corporels 11, un coussin absorbant 12 fixé sur la couche externe 11, par exemple par collage, une bande de zone d'entre-jambes en matériau selon l'invention 1 et un voile de surface 13 en non-tissé classique. Le voile de surface est réuni à la couche externe imperméable 11 par tous moyens classiques tels que par des lignes de collage 14. Comme le montrent les figures, la bande de zone de zone d'entre-jambes constituée du matériau de la figure 1 est disposée entre le coussin absorbant 12 et le voile de surface en non-tissé classique 13. Dans cette réalisation, la nappe de fibres de type cardé 3 du matériau composite 1 se trouve disposée directement sur la surface interne du coussin 12, cependant que la couche en non-tissé 2 constitue la couche la plus interne de la bande de zone d'entre-jambes qui se trouve être en contact avec la surface externe du voile de surface en non-tissé classique 13. La bande de zone d'entre-jambes est réunie à la surface externe du voile en non-tissé classique 13 ou à la surface interne du coussin absorbant 12 par tous moyens classiques tels que par collage, thermoscellage, scellage aux ultrasons ou par aiguilletage. Comme indiqué précédemment, cette bande de zone d'entre-jambes en matériau composite selon l'invention peut être disposée soit sur la surface interne du voile de surface en non-tissé classique 13 soit entre ce voile de surface 13 et le coussin absorbant 12. Toutefois, dans tous les cas, la couche en non-tissée 2 du matériau composite doit constituer la couche la plus interne de la bande de zone d'entre-jambes.

On réalise ainsi un article d'hygiène absorbant selon l'invention qui possède des propriétés de douceur, vitesse de traversée des fluides corporels, et de barrière au remouillage améliorées.

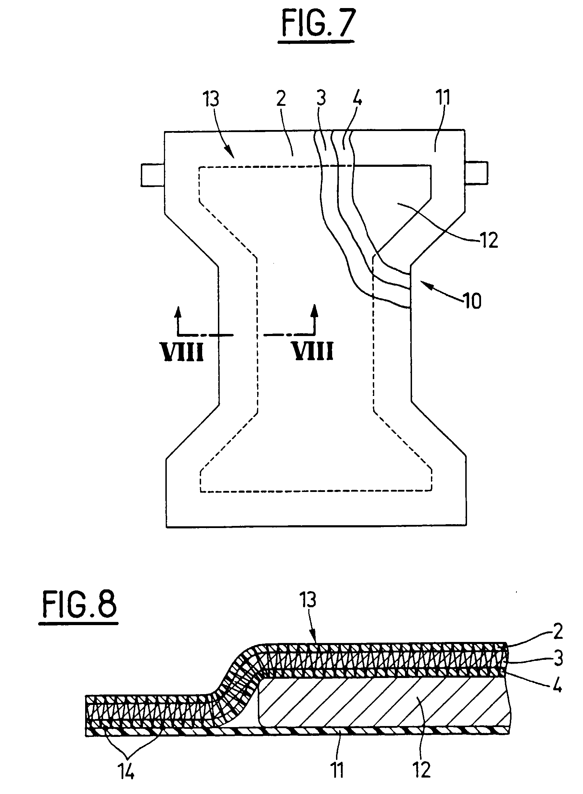

En se référant aux figures 7 et 8, on a représenté un article d'hygiène absorbant, tel qu'une couche-culotte, comprenant un voile de surface constitué par un matériau composite selon l'invention tel que représenté à la figure 2. Comme précédemment, l'article d'hygiène absorbant comprend une couche externe en matériau imperméable aux fluides corporels 11, un coussin absorbant 12 et un voile de surface 13 constitué du matériau composite selon l'invention décrit en liaison avec la figure 2. La couche externe imperméable 11, le coussin 12 et le voile de surface 13 ont une forme semblable à celle décrite en liaison avec les figures 3 et 4. Dans ce cas, le voile de surface est disposé de telle sorte que la couche en non-tissé de plus faible grammage 4 constitue la couche la plus externe du voile de surface, cependant que la couche en non-tissé de plus fort grammage 2 constituera la couche la plus interne du voile de surface 13 destiné à venir en contact avec le corps de l'utilisateur.

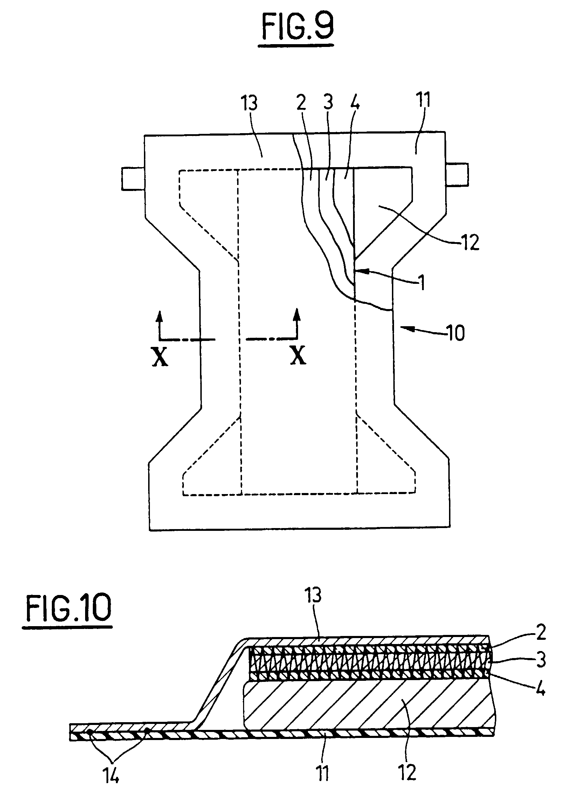

En se référant maintenant aux figures 9 et 10 on a représenté un article d'hygiène absorbant, tel qu'une couche-culotte 10, analogue à l'article d'hygiène absorbant représenté aux figures 5 et 6 et comprenant en complément d'un voile de surface classique 13 une bande de zone d'entre-jambes constituée du matériau composite selon l'invention décrit en liaison avec la figure 2. La couche-culotte 10 comprend une couche externe imperméable aux fluides corporels 11, un coussin absorbant 12, une bande de zone d'entre-jambes de largeur semblable à la largeur de la zone d'entre-jambes du coussin et de longueur semblable à celle de ce coussin. et un voile de surface en non-tissé classique 13. Le coussin 12 est fixé à la couche externe 11 par tout moyen classique, par exemple par collage et le voile de surface en non-tissé classique 13 est lié à la couche externe 11 sur le pourtour du coussin absorbant 12 par tout moyen classique tel que par des lignes de collage 14. La bande de zone d'entre-jambes, qui dans la réalisation représentée est disposée entre le coussin 12 et le voile de surface classique 13 se compose comme décrit précédemment de deux couches de non-tissé 4 et 2 entre lesquelles se trouve la nappe de fibres de type cardé 3. La couche en non-tissé de plus faible grammage 4 se trouve directement sur la surface interne du coussin 12, cependant que la couche en non-tissé 2 de grammage supérieur constitue la couche la plus interne de la bande d'entre-jambes qui se trouve en contact avec la surface interne du voile en non-tissé classique 13. La bande de zone d'entre-jambes en matériau composite selon l'invention peut être liée au coussin absorbant 12 par tous moyens connus tels que par collage, thermoscellage, scellage aux ultrasons ou par aiguilletage, l'utilisation de la couche de non-tissé de plus faible grammage 4 permettant une meilleure fixation du coussin absorbant 12. Bien évidemment, comme précédemment, la bande de zone d'entre-jambes en matériau composite selon l'invention pourrait être disposée sur la surface interne du voile en non-tissé classique 13, et dans ce cas, la couche de non-tissé de plus faible grammage 4 constituera la couche la plus externe de la bande de zone d'entre-jambes qui sera liée à la surface interne du voile de surface classique 13, cependant que la couche en non-tissé de grammage supérieur constituera la couche la plus interne de la bande de zone d'entre-jambes destinée à entrer en contact avec le corps de l'utilisateur.

On a ainsi réalisé un article d'hygiène absorbant ayant des propriétés de douceur, vitesse de traversée des fluides corporels et de barrière au remouillage améliorées.

Les exemples suivants non limitatifs, mettent en évidence les avantages obtenus en utilisant le matériau composite de l'invention comme voile de surface dans des couches-culottes.

### Exemple comparatif A

On a déterminé la vitesse de transpercement et la résistance au remouillage d'une couche-culotte du commerce (Peaudouce Action Girl® correspondant à la taille 8-18 kg, de poids total 59 g, ce coussin absorbant ayant un poids de 48 g dont 4,6 g de matériau superabsorbant) et qui comporte un voile de surface classique en non-tissé de fibres de polypropylène de type lié au filage (non-tissé "spun") ayant un grammage de 20 g/m². Les résultats des essais sont donnés dans le tableau 1.

### Exemple comparatif B

On a déterminé la vitesse de transpercement et la résistance au remouillage d'une autre couche-culotte du commerce, de taille identique à celle de l'exemple comparatif A et comportant un voile de surface constitué par une couche de base en non-tissé de fibres de polypropylène de type lié au filage (non-tissé "spun") ayant un grammage de 30 g/m² et d'une bande de zone d'entre-jambes en non-tissé de fibres majoritairement de polyester de type lié par traversée d'air (air-through bonded) ayant un grammage de 52 g/m², soit un grammage total de 82 g/m². Les résultats des essais sont donnés dans le tableau 1.

### Exemple comparatif C

On a remplacé le voile de surface de la couche-culotte de l'exemple comparatif A par le voile de surface de la couche-culotte de l'exemple comparatif B et on a mesuré la vitesse de transpercement et la résistance au remouillage de l'article ainsi obtenu. Les résultats sont donnés dans le tableau 1.

### Exemple comparatif D

On a ajouté au voile de surface de la couche-culotte de l'exemple comparatif A la bande de zone d'entre-jambes de la couche-culotte de l'exemple comparatif 2, c'est-à-dire la bande en matériau non-tissé de type lié par traversée d'air ayant un grammage de 52 g/m², et on a mesuré la vitesse de transpercement et la résistance au remouillage. Les résultats sont donnés dans le tableau 1.

### Exemple comparatif E

On a ajouté au voile de surface de la couche-culotte de l'exemple comparatif A quatre couches d'un non-tissé de fibres de polypropylène de type lié au filage (spun) ayant chacune un grammage de 20 g/m². On a mesuré la vitesse de transpercement et la résistance au remouillage de l'article obtenu. Les résultats sont donnés dans le tableau 1.

### Exemple 1

On a remplacé le voile de surface de la couche-culotte de l'exemple comparatif A par un voile de surface constitué d'un matériau composite selon l'invention. Dans cet exemple, le matériau composite selon l'invention était constitué d'une première couche d'un non-tissé de fibres de polyéthylène de type lié au filage (spun) d'un grammage de 20 g/m² de la Société COROVIN liée par aiguilletage à une nappe de fibres de polyester de type cardé de 6,6 deniers ayant un grammage de 50 g/m², soit un grammage total de 70 g/m². La densité d'aiguilletage était de 20 coups d'aiguille par cm² et par face et la masse volumique du matériau obtenu était de 150 kg/m³. La nappe de fibres de polyester de type cardé était disposée directement sur le coussin absorbant. On a mesure la vitesse de transpercement et la résistance au remouillage de l'article obtenu. Les résultats sont donnés dans le tableau 1.

### Exemple 2

On remplace le voile de surface de la couche-culotte de l'exemple comparatif B par un voile en non-tissé composite selon l'invention identique à celui de l'exemple 1, dont la nappe de fibres de type cardé se trouve alors directement en contact avec le coussin absorbant de la couche-culotte. On a mesuré la vitesse de transpercement et la résistance au remouillage de l'article obtenu. Les résultats sont donnés dans le tableau 1.

### Exemple 3

On retire la bande de non-tissé de 52 g/m² de la couche-culotte de l'exemple comparatif B et on la remplace par le non-tissé composite de l'exemple 1, avec la nappe de fibres de type cardé en contact avec la couche de base en non-tissé de 30 g/m² du voile de surface initial de la couche-culotte. On a mesuré la vitesse de transpercement et la résistance au remouillage de l'article obtenu. Les résultats sont donnés dans le tableau 1.

### Exemple 4

On a ajouté sur le voile de surface de la couche-culotte de l'exemple comparatif A un voile constitué d'un matériau non-tissé composite selon l'invention comportant une première couche d'un non-tissé de fibres de polyéthylène de type lié au filage (spun) de 20 g/m², une nappe de fibres de polyester de type cardé de 50 g/m² analogues à celles de l'exemple 1 et une seconde couche d'un non-tissé de fibres de polypropylène de type lié au filage (spun) de 17 g/m², les trois couches étant liées par aiguilletage. La densité d'aiguilletage était de 20 coups d'aiguille par cm² et par face. Le non-tissé composite selon l'invention est disposé avec la seconde couche de non-tissé en contact avec le voile de surface initial. On a mesuré la vitesse de transpercement et la résistance au remouillage de l'article obtenu. Les résultats sont donnés dans le tableau 1.

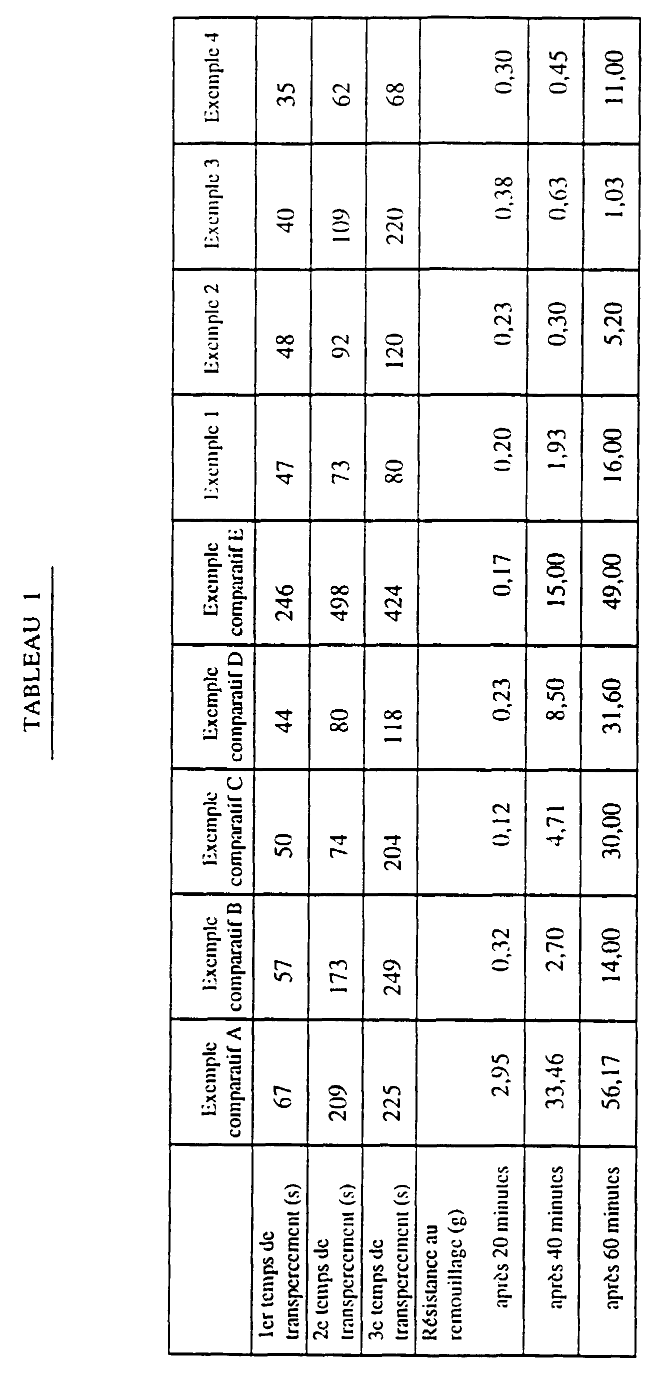

Les temps de transpercement et la résistance au remouillage ont été déterminés de la façon suivante :

Les produits finis sont conditionnés à 23°C et 50% d'humidité relative pendant 24 heures avant les essais.

On place au centre du voile de surface de l'article testé une plaque en plexiglas ® de 7x7 cm perforée en son centre. On verse dans l'orifice de la plaque 100 cm³ d'une solution saline à 9 g/l de chlorure de sodium dans de l'eau distillée au moyen d'une ampoule à décanter en réglant l'écoulement de l'ampoule pour avoir un niveau constant haut dans l'orifice de la plaque. On mesure le temps écoulé entre le début de l'introduction de la solution saline et l'instant auquel la solution saline a disparu dans l'article. Le temps mesuré constitue le premier temps de transpercement.

On pèse alors six papiers filtres DIMAR ED 939 ® découpés en carrés de 10,2 x 10,2 cm. On place ensuite, après avoir ôté la plaque perforée, sur le voile de surface de l'article testé, un poids de 3,5 kg de 10,2 x 10.2 cm pendant 10 minutes. Une fois les 10 minutes écoulées, on place sous le poids les six papiers filtres et on laisse encore pendant 10 minutes. A la fin de cette période on retire le poids et les papiers filtres. On pèse les papiers filtres. La différence de poids, en grammes, entre la première et la seconde pesée donne une mesure de la résistance au remouillage après 20 minutes.

On recommence deux fois la procédure ci-dessus avec le même article, en utilisant respectivement 24 filtres après la deuxième addition de solution saline et 30 filtres après la troisième addition. On obtient ainsi les deuxième et troisième temps de transpercement ainsi que la résistance au remouillage après 40 et 60 minutes.

Comme le montre clairement les exemples du tableau 1 ci-dessus, l'utilisation du voile de surface en non-tissé composite selon l'invention en remplacement ou en complément d'un voile de surface classique résulte en une combinaison particulièrement avantageuse d'un temps de transpercement court et d'une meilleure résistance au remouillage. En particulier l'exemple 1 présente un temps de transpercement au troisième essai environ 60% plus court que celui de l'exemple comparatif C et une amélioration de la résistance au remouillage après 60 minutes d'environ 47%.

Une comparaison des résultats obtenus pour l'exemple 2 avec ceux de l'exemple comparatif B, montre qu'en substituant un voile de surface selon l'invention du voile de surface initial on obtient une amélioration des deuxième et troisième temps de transpercement d'environ 50% et de la résistance au remouillage après 60 minutes d'environ 63%.

La comparaison des résultats obtenus à l'exemple 4 avec ceux de l'exemple 1 montre que l'utilisation d'un non-tissé composite selon l'invention comportant une deuxième couche de non-tissé en complément du voile de surface classique ne nuit pas au temps de transpercement tout en améliorant la résistance au remouillage après 60 minutes d'environ 30%.

Enfin, une comparaison des exemples selon l'invention avec l'exemple comparatif E montre que le grammage total du voile de surface n'influe pas sur les résultats. Ainsi, on peut considérer que le voile de surface de l'exemple 4 est constitué de quatre couches pour un grammage total de 107 g/m² et le voile de surface de l'exemple comparatif E de cinq couches pour un grammage total de 100 g/m². La comparaison des résultats obtenus montrant pour l'exemple comparatif une augmentation de 500 à 600% des temps de transpercement et une détérioration de 345% de la résistance au remouillage après 60 minutes.

On a ainsi réalisé selon l'invention un matériau composite non-tissé particulièrement utile dans la fabrication de voiles de surface pour articles d'hygiène absorbant.

## Revendications

1. Matériau non-tissé composite perméable aux fluides corporels ayant une vitesse de traversée par les liquides et une résistance au remouillage améliorées, caractérisé en ce qu'il comprend au moins une couche composée d'un non-tissé (2) perméable aux fluides corporels, et sur cette première couche, une nappe de fibres de type cardé (3), perméable aux fluides corporels, la nappe de fibres de type cardé étant liée à la première couche par un aiguilletage (5).

2. Matériau non-tissé composite selon la revendication 1 caractérisé en ce qu'il comprend une seconde couche de non-tissé (4), la nappe de fibres de type cardé (3) étant disposée entre la première et seconde couches de non-tissé (2, 4), la seconde couche de non-tissé (4) ayant un grammage inférieur à la première couche de non-tissé, les couches de non-tissé (2, 4) et la nappe de fibres de type cardé (3) étant liée par un aiguilletage (5).

3. Matériau non-tissé composite selon l'une quelconque des revendications précédentes caractérisé en ce que les première et deuxième couches de non-tissé sont constituées par des fibres textiles naturelles ou synthétiques choisies parmi les fibres de cellulose, viscose, polyester, polyéthylène, polypropylène, nylon ou copolymères d'éthylène-propylène.

4. Matériau non-tissé composite selon l'une quelconque des revendications précédentes caractérisé en ce que la nappe de fibres de type cardé (3) est constituée de fibres textiles synthétiques choisies parmi les fibres de polyester, polyéthylène, polypropylène, nylon ou copolymères d'éthylène-propylène.

5. Matériau non-tissé composite selon l'une quelconque des revendications précédentes caractérisé en ce que la première couche de non-tissé (2) a un grammage compris entre 10 et 30 g/m².

6. Matériau non-tissé composite selon l'une quelconque des revendications 2 à 5 caractérisé en ce que la deuxième couche de non-tissé (4) a un grammage inférieur à 20 g/m².

7. Matériau non-tissé composite selon l'une quelconque des revendications précédentes caractérisé en ce que la nappe de fibres de type cardé (3) à un grammage compris entre 20 et 60 g/m².

8. Matériau non-tissé composite selon la revendication 7 caractérisé en ce que la nappe de fibres de type cardé (3) a un grammage de 50 g/m².

9. Matériau non-tissé composite selon l'une quelconque des revendications précédentes, caractérisé en ce que la nappe de fibres de type cardé (3) a un denier compris entre 3 et 13.

10. Article d'hygiène absorbant comprenant une couche externe imperméable aux fluides corporels (11), un coussin absorbant (12) perméable aux fluides corporels et fixé à la couche externe (11), et un voile de surface (13) perméable aux fluides corporels et fixé à la couche externe (11) caractérisé en ce que le voile de surface est constitué par un matériau non-tissé composite selon l'une quelconque des revendications précédentes, la nappe de fibres de type cardé (3), ou la seconde couche de non-tissé (4), lorsqu'elle est présente, constituant la couche la plus externe du voile de surface, la première couche de non-tissé (2) constituant la couche la plus interne du voile de surface destinée à venir en contact avec le corps de l'utilisateur.

11. Article d'hygiène absorbant comprenant une couche externe en matériau imperméable aux fluides corporels (11), un coussin absorbant (12) perméable aux fluides corporels fixé à la couche externe (11) et comportant deux parties d'extrémité opposées élargies et une zone d'entre-jambes plus étroite, une bande de zone d'entre-jambes perméable aux fluides corporels, de largeur analogue à la zone d'entre-jambes du coussin et de longueur analogue au coussin, et un voile de surface (13) perméable aux fluides corporels et fixé à la couche externe (11) caractérisé en ce que la bande de zone d'entre-jambes est constituée du matériau non-tissé composite (1) selon l'une quelconque des revendications 1 à 9, la nappe de fibres de type cardé (3), ou la seconde couche de non-tissé de plus faible grammage (4), lorsqu'elle est présente, constituant la couche la plus externe de la bande de zone d'entre-jambes.

12. Articlc d'hygiène absorbant selon la revendication 11 caractérisé en ce que la bande de zone d'entre-jambes est disposée sur la surface interne du voile de surface (13).

13. Article d'hygiène absorbant selon la revendication 11 caractérisé en ce que la bande de zone d'entre-jambes est disposée entre le coussin absorbant (12) et le voile de surface (13).

14. Article d'hygiène absorbant selon la revendication 12 ou 13 caractérisé en ce que la nappe de fibres de type cardé (3), ou la deuxième couche de non-tissé de plus faible grammage (4), lorsqu'elle est présente, est liée à la surface interne du voile de surface (13) ou du coussin absorbant (12) par collage, thermoscellage, scellage aux ultrasons ou par aiguilletage.

15. Article d'hygiène absorbant comprenant un voile de surface (13) constitué d'un matériau non-tissé composite selon l'une quelconque des revendications 1 à 9.

16. Article d'hygiène absorbant comprenant un voile de surface (13) et une bande de zone d'entre-jambes, caractérisé en ce que la bande de zone d'entrejambes est constituée d'un matériau non-tissé composite selon l'une quelconque des revendications 1 à 9.

## Patentansprüche

1. Vliesstoff-Verbundmaterial, das für Körperflüssigkeiten durchlässig ist und verbesserte Werte bei der Durchdringungsgeschwindigkeit der Flüssigkeiten sowie bei der Durchfeuchtungsbeständigkeit aufweist,
**dadurch gekennzeichnet**, daß
es mindestens eine Schicht (2) aus einem für Körperflüssigkeiten durchlässigen Vliesstoff und auf dieser ersten Schicht ein für Körperflüssigkeiten durchlässiges Vlies (3) aus Fasern vom kardierten Typ umfaßt, wobei das Vlies aus Fasern vom kardierten Typ mit der ersten Schicht durch eine Nadelung (5) verbunden ist.

2. Vliesstoff-Verbundmaterial gemäß Anspruch 1,
**dadurch gekennzeichnet,** daß
es eine zweite Vliesstoffschicht (4) umfaßt, wobei das Vlies (3) aus den Fasern vom kardierten Typ zwischen den ersten und zweiten Vliesstoffschichten (2, 4) angeordnet ist, die zweite Vliesstoffschicht (4) ein geringeres Grammgewicht als die erste Vliesstoffschicht aufweist und die Vliesstoffschichten (2, 4) und das Vlies (3) aus den Fasern vom kardierten Typ durch eine Nadelung (5) verbunden sind.

3. Vliesstoff-Verbundmaterial gemäß jedem der vorhergehenden Ansprüche,
**dadurch gekennzeichnet**, daß
die ersten und zweiten Vliesstoffschichten aus natürlichen oder synthetischen Textilfasern zusammengesetzt sind, die aus Fasern aus Zellulose, Viskose, Polyester, Polyethylen, Polypropylen, Nylon oder aus Copolymeren aus Ethylen-Propylen ausgewählt sind.

4. Vliesstoff-Verbundmaterial gemäß jedem der vorhergehenden Ansprüche,
**dadurch gekennzeichnet**, daß
das Vlies (3) aus Fasern vom kardierten Typ aus synthetischen Textilfasern zusammengesetzt ist, die aus Fasern aus Polyester, Polyethylen, Polypropylen, Nylon oder aus Copolymeren von Ethylen-Propylen ausgewählt sind.

5. Vliesstoff-Verbundmaterial gemäß jedem der vorhergehenden Ansprüche,
**dadurch gekennzeichnet**, daß
die erste Vliesstoffschicht (2) ein Grammgewicht von 10 bis 30 g/m² aufweist.

6. Vliesstoff-Verbundmaterial gemäß jedem der vorhergehenden Ansprüche 2 bis 5,
**dadurch gekennzeichnet**, daß
die zweite Vliesstoffschicht (4) ein Grammgewicht von weniger als 20 g/m² aufweist.

7. Vliesstoff-Vebundmaterial gemäß jedem der vorhergehenden Ansprüche,
**dadurch gekennzeichnet**, daß
das Vlies (3) aus Fasern vom kardierten Typ ein Grammgewicht von 20 bis 60 g/m² aufweist.

8. Vliesstoff-Verbundmaterial gemäß Anspruch 7,
**dadurch gekennzeichnet**, daß
das Vlies (3) aus Fasern vom kardierten Typ ein Grammgewicht von 50 g/m² aufweist.

9. Vliesstoff-Verbundmaterial gemäß jedem der vorhergehenden Ansprüche,
**dadurch gekennzeichnet**, daß
das Vlies (3) aus Fasern vom kardierten Typ zusammengesetzt ist, die einen Denier-Wert von 3 bis 13 aufweisen.

10. Absorbierender Hygieneartikel, umfassend eine für Körperflüssigkeiten undurchlässige Außenschicht (11), ein für Körperflüssigkeiten durchlässiges absorbierendes Kissen (12), das an die Außenschicht (11) fixiert ist, sowie eine für Körperflüssigkeiten durchlässige Oberflächenschleierstoffbahn (13), die ebenfalls an die Außenschicht (11) fixiert ist,
**dadurch gekennzeichnet**, daß
der Oberflächenschleierstoff aus einem Vliesstoff-Verbundmaterial gemäß jedem der vorhergehenden Ansprüche zusammengesetzt ist, wobei das Vlies (3) aus den Fasern vom kardierten Typ oder die zweite Vliesstoffschicht (4), falls vorhanden, die äußerste Schicht der Oberflächenschleierstoffbahn und die erste Vliesstoffschicht (2) die innerste Schicht der Oberflächenschleierstoffbahn darstellen, die dazu bestimmt ist, in Kontakt mit dem Körper des Benutzers zu gelangen.

11. Absorbierender Hygieneartikel, umfassend eine Außenschicht (11) aus für Körperflüssgikeiten undurchlässigem Material, ein für Körperflüssigkeiten durchlässiges Kissen (12), das an die Außenschicht (11) fixiert ist und zwei gegenüberliegende vergrößerte Außenteilstücke und eine engere Zwischenbeinzone aufweist, eine für Körperflüssigkeiten durchlässige Binde für die Zwischenbeinzone mit einer Größe, die der Zwischenbeinzone des Kissens gleicht, und einer Länge, die dem Kissen gleicht, sowie eine Oberflächenschleierstoffbahn (13), die für Körperflüssigkeiten durchlässig und an die Außenschicht (11) fixiert ist,
**dadurch gekennzeichnet**, daß
die für die Zwischenbeinzone vorgesehene Binde aus dem Vliesstoff-Verbundmaterial (1) gemäß jedem der Ansprüche 1 bis 9 zusammengesetzt ist, wobei das Vlies (3) aus den Fasern vom kardierten Typ oder die zweite Vliesstoffschicht (4) mit dem geringeren Grammgewicht, falls vorhanden, die äußerste Schicht der für die Zwischenbeinzone vorgesehenen Binde darstellen.

12. Absorbierender Hygieneartikel gemäß Anspruch 11,
**dadurch gekennzeichnet**, daß
die Binde für die Zwischenbeinzone auf der inneren Oberfläche der Oberflächenschleierstoffbahn (13) angeordnet sit.

13. Absorbierender Hygieneartikel gemäß Anspruch 11,
**dadurch gekennzeichnet**, daß
die Binde für die Zwischenbeinzone zwischen dem absorbierenden Kissen (12) und der Oberflächenschleierstoffbahn (13) angeordnet ist.

14. Absorbierender Hygieneartikel gemäß Anspruch 12 oder 13,
**dadurch gekennzeichnet**, daß
das Vlies (3) aus den Fasern vom kardierten Typ oder die zweite Vliesstoffschicht (4) mit dem geringeren Grammgewicht, falls vorhanden, mit der inneren Oberfläche der Oberflächenschleierstoffbahn (13) oder des absorbierenden Kissens (12) durch Kleben, Thermoversiegeln, Versiegeln mit Ultraschall oder durch Nadelung verbunden sind.

15. Absorbierender Hygieneartikel, umfassend eine Oberflächenschleierstoffbahn (13) aus einem Vliesstoff-Verbundmaterial gemäß jedem der Ansprüche 1 bis 9.

16. Absorbierender Hygieneartikel, umfassend eine Oberflächenschleierstoffbahn (13) sowie eine für die Zwischenbeinzone vorgesehene Binde,
**dadurch gekennzeichnet**, daß
die für die Zwischenbeinzone vorgesehene Binde aus einem Vliesstoff-Verbundmaterial gemäß jedem der Ansprüche 1 bis 9 zusammengesetzt ist.

## Claims

1. Composite nonwoven material permeable to body fluids and having an improved rate of passage by the liquids and improved resistance to rewetting, characterized in that it comprises at least one layer made up of a nonwoven (2) which is permeable to body fluids and, on this first layer, a sheet of fibres of carded type (3), permeable to body fluids, the sheet of fibres of carded type being bonded to the first layer by a needling (5).

2. Composite nonwoven material according to Claim 1, characterized in that it comprises a second nonwoven layer (4), the sheet of fibres of carded type (3) being arranged between the first and second nonwoven layers (2, 4), the second nonwoven layer (4) having a weight per unit area lower than the first nonwoven layer, the nonwoven layers (2, 4) and the sheet of fibres of carded type (3) being bonded by a needling (5).

3. Composite nonwoven material according to either of the preceding claims, characterized in that the first and second nonwoven layers consist of natural or synthetic textile fibres chosen from cellulose, viscose, polyester, polyethylene, polypropylene, nylon or ethylene-propylene copolymer fibres.

4. Composite nonwoven material according to any one of the preceding claims, characterized in that the sheet of fibres of carded type (3) consists of synthetic textile fibres chosen from polyester, polyethylene, polypropylene, nylon or ethylene-propylene copolymer fibres.

5. Composite nonwoven material according to any one of the preceding claims, characterized in that the first nonwoven layer (2) has a weight per unit area of between 10 and 30 g/m².

6. Composite nonwoven material according to any one of Claims 2 to 5, characterized in that the second nonwoven layer (4) has a weight per unit area lower than 20 g/m².

7. Composite nonwoven material according to any one of the preceding claims, characterized in that the sheet of fibres of carded type (3) has a weight per unit area of between 20 and 60 g/m².

8. Composite nonwoven material according to Claim 7, characterized in that the sheet of fibres of carded type (3) has a weight per unit area of 50 g/m².

9. Composite nonwoven material according to any one of the preceding claims, characterized in that the sheet of fibres of carded type (3) has a denier of between 3 and 13.

10. Absorbent article of hygiene comprising an outer layer which is impervious to body fluids (11), an absorbent pad (12) which is permeable to body fluids and secured to the outer layer (11) and a surface web (13) which is permeable to body fluids and secured to the outer layer (11), characterized in that the surface web consists of a composite nonwoven material as claimed in any one of the preceding claims, the sheet of fibres of carded type (3) or the second nonwoven layer (4), when it is present, forming the outermost layer of the surface web, the first nonwoven layer (2) forming the innermost layer of the surface web intended to come into contact with the user's body.

11. Absorbent article of hygiene comprising an outer layer made of material which is impervious to body fluids (11), an absorbent pad (12) which is permeable to body fluids, secured to the outer layer (11) and comprising two widened opposed end parts and a narrower crotch region, a crotch region strip which is permeable to body fluids, of width similar to the crotch region of the pad and of length similar to the pad, and a surface web (13) which is permeable to body fluids and secured to the outer layer (11), characterized in that the crotch region strip consists of the composite nonwoven material (1) according to any one of Claims 1 to 9, the sheet of fibres of carded type (3) or the second nonwoven layer of lower weight per unit area (4), when it is present, forming the outermost layer of the crotch region strip.

12. Absorbent article of hygiene according to Claim 11, characterized in that the crotch region strip is arranged on the inner surface of the surface web (13).

13. Absorbent article of hygiene according to Claim 11, characterized in that the crotch region strip is arranged between the absorbent pad (12) and the surface web (13).

14. Absorbent article of hygiene according to Claim 12 or 13, characterized in that the sheet of fibres of carded type (3) or the second nonwoven layer of lower weight per unit area (4), when it is present, is bonded to the inner surface of the surface web (13) or of the absorbent pad (12) by adhesive bonding, heat-sealing, ultrasonic sealing or by needling.

15. Absorbent article of hygiene comprising a surface web (13) consisting of a composite nonwoven material according to any one of Claims 1 to 9.

16. Absorbent article of hygiene comprising a surface web (13) and a crotch region strip, characterised in that the crotch region strip consists of a composite nonwoven material according to any one of Claims 1 to 9.
